# EUROPEAN PATENT APPLICATION

(11) **EP 1 811 414 A2**
(43) Date of publication of application: **25.07.2007**
(21) Application number: 07000862.8
(22) Date of filing: 17.01.2007
(51) Int. Cl.: G06F 19/00, G06Q 50/00

(54) **Procedure to actuate the monitoring and certification of the freezing and defrosting cycle of blood and hemocomponents**

(30) Priority: 20.01.2006 IT MO20060019
(71) Applicant: EXPERTMED S.R.L., 37139 Verona VR (IT)
(72) Inventor: Sgarbi, Massimo, 37139 Verona VR (IT)
(74) Representative: Cerreta, Caterina

(57) **Abstract**

The invention falls into the field of procedures for the treatment of data inherent to a biological liquid freezing and/or defrosting process. A procedure envisages, at the least, the following steps: data detection and recording; transfer of the said data to a computer; processing, analysis and archiving of the said data, and displaying and printing out of the said data.

## Description

The invention relates to a procedure for the monitoring and certification of the freezing and defrosting cycle of blood or hemocomponents.

It is known that current legislation concerning the treatment of blood and the products derived therefrom, which shall be referred to hereinafter as "biological liquid", requires certification of the that freezing of the said biological liquid contained within the relative container bags has taken place.

The freezing required is represented by the reaching of the temperature -30°C within a maximum time of one hour from the beginning of the insertion of the bags into the relative freezing device.

Legislation in Italy provides for the presence, throughout the entire national territory, of certified companies appointed by the Ministry of Health to collect, from hospital structures, plasma and other biological products frozen with appropriate freezing processes for subsequent industrial processing; the aforesaid companies then take measures to establish a classification of the quality of the freezing that has taken place by means of their own protocols.

Naturally, this is a classification which cannot be submitted to certification involving the use of absolute parameters since the collection companies do not know how the biological liquid has effectively been frozen at the various hospitals, since there lacks a way of tracing the data relating to the correct freezing cycle attained.

In fact, for example, if a hospital structure utilises a highly efficient freezing device but without any direct monitoring system, one can only assume that, after the biological liquid has been inside the aforesaid device for an hour, the heart of the product inside the bag has reached the required temperature of-30°C. As appears evident, this valuation of whether the freezing of all the biological product contained in the bags has taken place has a rather empirical nature, featuring margins of error, which is why it is not certifiable, and all this has negative repercussions on the valuation effected by the aforesaid collection companies.

It therefore appears evident that the current sources of uncertainty as to whether the freezing/defrosting process applied to the biological product has taken place according to the parameters set by the health authorities constitute the major drawback in the effecting of the certification of the aforesaid process.

The aim of the present invention is to overcome the aforesaid drawback.

In particular, the procedure suitable for the monitoring and certification of the freezing and defrosting cycle of the blood or hemocomponents in question in the present invention is
characterised by the fact that the said procedure is composed of, at least, the following succession of steps:
- detection and recording of the data collected from the moment of activation of the said procedure, on the basis of the programming received from dedicated software;
- transfer, to an electronic elaboration element, of all the data collected at the end of each freezing/defrosting process;
- processing, analysis and archiving, by means of the use of the dedicated software, of all the data collected at the end of each freezing/defrosting process;
- displaying and printing out of the aforesaid data.

This and other characteristics will better emerge from the detailed description that follows of a preferred embodiment.

A specially dedicated electronic system is suitable to program both the switching on and off of a collection device for the data (such as, for example, temperature, time intervals, alarms) and the duration in time of the data acquisition.

The procedure designed to lead to the certification of the data relating to the freezing/defrosting process applied to the biological liquid permits, from the moment of activation of the aforesaid dedicated electronic system, the detection and recording of the temperatures by following programming set previously by the operator.

The freezing/defrosting process proceeds until its completion, at which point the procedure provides for the transfer of the data collected to an electronic elaboration unit, for example, a computer. The said transfer can take place, irrespectively, via cable or via ether.

The procedure in question in the present invention envisages that all the data relating to each freezing/defrosting process effected is subsequently elaborated, analysed and archived by the aforesaid electronic elaboration unit.

The procedure subsequently envisages the displaying and printing out of all the data processed previously.

It is opportune to note that for the implementation of the procedure in question in the present invention both flexible bags and rigid containers are employed, both made of a plastic material, capable of withstanding at least 50 freezing/defrosting cycles at a freezing temperature of at least -30°C. The said flexible bags and rigid containers can present different capacities, ranging from 100 to 1000 ml.

The procedure described envisages the use of a liquid solution of a synthetic type endowed with chemical/physical characteristics during freezing/defrosting which are analogous to the biological liquid to be submitted to the thermal treatment. The said analogy should be considered in the sense that the liquid solution utilised has a freezing curve which entirely comprises the freezing curve of the fresh biological product.

The procedure in question in the present invention envisages that the flexible bags or rigid containers employable are all endowed with a thermometer equipped with temperature sensors; the thermometers utilised are either of the type which are fully immersed in the heart of the bag or container or of the external type, the said thermometers being known as "data loggers".

As far as the temperature sensors are concerned, these may be of the type which are immersed or of the type which is external to the flexible bag or rigid container.

Therefore the procedure in question in the present invention can utilise various combinations of elements suitable to detect and measure the temperature of the biological liquid thanks to the aforesaid analogy. One combination is constituted of the use of a an immersion thermometer in which the temperature sensors are an integral part thereof. A further combination is constituted of an external thermometer and a temperature sensor, for example a probe which is immersed in the said synthetic-type liquid solution. A still further combination is constituted of an external thermometer fitted with a sensor element which is external to the flexible bag or rigid container.

The main advantage of the procedure in question in the present invention is constituted of the possibility of effecting the certification of data regarding the results obtained from a specific thermal treatment to which a flexible bag or rigid container containing biological liquid is submitted.

## Claims

1. A procedure suitable for the monitoring and certification of the freezing and defrosting cycle of blood or hemocomponents, for example, plasma, platelets, erythrocytes, white globules, **characterised by** the fact that the said procedure is comprised of, at least, the following succession of steps:
- detection and recording of the data collected from the moment of activation of the said procedure, on the basis of the programming received from dedicated software;
- transfer, to an electronic elaboration element, of all the data collected at the end of each freezing/defrosting process;
- elaboration, analysis and archiving, by means of the use of the dedicated software, of all the data collected at the end of each freezing/defrosting process;
- displaying and printing out of the aforesaid data.

2. A procedure according to claim 1, **characterised by** the fact that the transfer of data to the electronic processing element is of the 'via cable' type.

3. A procedure according to claim 1 **characterised by** the fact that the transfer of data to the electronic processing element is of the 'via ether' type.

4. A procedure according to the previous claims **characterised by** the fact that, in order to contain a liquid solution of a synthetic type to be submitted to thermal treatment, flexible bags made of a plastic material are utilised which are capable of withstanding at least 50 freezing/defrosting cycles at a freezing temperature of at least -30°C.

5. A procedure according to the previous claims **characterised by** the fact that in order to contain a liquid solution of a synthetic type to be submitted to thermal treatment, rigid containers made of a plastic material are utilised which are capable of withstanding at least 50 freezing/defrosting cycles at a temperature of at least -30°C.

6. A procedure according to the previous claims, **characterised by** the fact that a liquid solution is utilised which is of a synthetic type endowed with chemical/physical characteristics during freezing/defrosting which are analogous to the biological liquid to be checked.

7. A procedure according to claims 1, 4 and 5, **characterised by** the fact that flexible bags and rigid containers are utilised, each of which is endowed with a temperature measuring element equipped with temperature sensors.

8. A procedure according to claims 1 and 7 **characterised by** the fact that the temperature measuring elements utilised are of the immersion type or of the type which is external to the flexible bag and to each rigid container.

9. A procedure according to claims 1 and 7, **characterised by** the fact that the temperature sensors are external or internal to each flexible bag and to each rigid container.

10. A procedure according to the previous claims **characterised by** the fact that the said procedure is applicable to flexible bags and to rigid containers, each one having different capacities, ranging from 100 to 1000 ml.
